# EUROPEAN PATENT APPLICATION

(11) **EP 2 220 998 A1**
(43) Date of publication of application: **25.08.2010**
(21) Application number: 08859490.8
(22) Date of filing: 26.11.2008
(51) Int. Cl.: A61B 5/145, A61B 5/00, H05K 5/02

(54) **MEDICAL DEVICE**

(30) Priority: 10.12.2007 JP 2007318327
(71) Applicant: ARKRAY, Inc., Minami-ku Kyoto-shi Kyoto 601-8045 (JP)
(72) Inventor: NISHIMURA, Hideki, Kyoto-shi Kyoto 601-8045 (JP); KUBO, Masayuki, Kyoto-shi Kyoto 601-8045 (JP)
(74) Representative: Mathys & Squire LLP
(86) International application number: PCT/JP2008/003474
(87) International publication number: WO 2009/075066

(57) **Abstract**

In a medical device (1) that measures a condition of a living body, a recess (8) is formed on the exterior (surfaces (6) and (7)) of its casing (2). An attachment member (3) is attached to the inside of the recess (8). In the case where a display screen (4) that displays a measurement result is provided on the casing (2), it is preferable that the surfaces (6) and (7) are adjacent to a surface (5) on which the display screen (4) is provided. Preferably, the attachment member (3) includes an adhesive material layer, a print layer, and an anti-slip layer that is formed of a resin material or a rubber material, and the adhesive material layer, the print layer, and the anti-slip layer are stacked in sequence.

## Description

### Technical Field

The present invention relates to medical devices, and particularly to a portable medical device.

### Background Art

Recently, the size reduction of medical devices has advanced, and the size of glucose meters, sphygmomanometers, pulse rate meters and the like, for example, has been reduced to such a degree that they can be portable. Such medical devices are often carried around by patients, medical personnel and the like, and their frequency of use is high. In particular, glucose meters are essential medical devices for diabetic patients, the frequency of carrying and the frequency of use of glucose meters are both very high, and they can be considered as highly personal.

Furthermore, among the users of such highly personal devices, there is a desire to customize the appearance of the devices according to their preferences, thereby expressing their individuality. Protective covers that enable the users to customize the appearance of medical devices according to their preference have been proposed recently (for example, see Patent document 1).

Specifically, Patent document 1 proposes a protective cover on which a pattern or a design is printed. The user can customize the appearance of the device by using a protective cover provided with a design of his or her preference. As a result, it is also possible to prevent the user from losing interest in that particular device model, which encourages the user to use the same device model for a long time. Furthermore, this promotes the extension in cycle life of the device, thereby making it possible to reduce the environmental load.
Patent document 1: JP2004-313269A (page 6, FIG. 6)

### Disclosure of Invention

### Problem to be Solved by the Invention

However, each time the user changes the protective cover to alter the appearance of the device, it is necessary to purchase a new protective cover, resulting in a large economic burden on the user. In addition, when a protective cover is attached to a medical device, the size of the medical device is increased by the size of the protective cover, which prevents the progress of size reduction of medical devices.

On the other hand, it is possible to alter the appearance at low cost without preventing the progress of size reduction, for example, by attaching a self-adhesive label to the casing of the device. However, the self-adhesive label may be easily detached by, for example, coming into contact with an external object. In that case, the aesthetic of the device is diminished and the user is required to perform troublesome work such as reattachment.

It is an object of the present invention to provide a medical device that can solve the above-described problem, and can easily be altered in its appearance at low cost without preventing the progress of size reduction.

### Means for Solving Problem

In order to achieve the above-described object, a medical device according to the present invention is a medical device that measures a condition of a living body, including a casing and an attachment member, wherein the attachment member is attached to an exterior of the casing. With the above-described feature, it is possible to alter the appearance of a medical device at low cost without using a protective cover as in conventional technologies. In this case, the progress of size reduction of medical devices will not be prevented. If the attachment member has a prominent design, then it is possible to prevent the medical device from being lost, even if it is reduced in size and thus more susceptible to be lost.

In the above-described medical device of the present invention, it is preferable that a recess is provided in the exterior of the casing, and the attachment member is attached to the inside of the recess. In this case, simply attaching the attachment member to the recess is sufficient, so that it is possible to alter the appearance easily. Furthermore, the attachment member is attached to the inside of the recess, and its edge does not easily come into contact with an external object. Accordingly, unlike the case where a self-adhesive label is simply attached, the attachment member does not easily become detached.

In the above-described medical device of the present invention, it is preferable that the casing is provided with a display screen that displays a measurement result, and the recess is provided in a surface adjacent to a surface where the display screen is provided. In this case, since a member having an anti-slipping effect is disposed at a position with which the hand of the user can easily come into contact, the occurrence of accidental dropping of the device by the user will be further suppressed.

In a preferred embodiment of the above-described medical device of the present invention, the recess is provided such that a gap is formed between an opening edge thereof and the attachment member. In this embodiment, when it is necessary to change the attachment member, the user can easily detach an old attachment member by inserting a finger or the like into the gap.

Furthermore, in the above embodiment, it is preferable that the medical device further includes a cover that closes the gap, the cover is fixed to the recess via an elastic body, and the elastic body is elastically deformed when the cover is pressed toward the inside of the recess by an external force. By doing so, the gap is closed when the detachment of the attachment member is not carried out, so that it is possible to suppress the occurrence of a situation where the attachment member is detached by coming into contact with an external object.

In another embodiment of the above-described medical device of the present invention, the casing is provided with a groove along an outer circumference of the recess. With the above-described embodiment, the attachment member can be pressed via the groove, and thus the attachment member is deformed. As a result, the contact area between the attachment member and the recess is reduced, which facilitates an operation for detaching the attachment member.

Furthermore, in another embodiment of the above-described medical device of the present invention, the recess is formed such that the bottom of a cross section thereof is V-shaped. In the above embodiment, the attachment member is attached in an elastically deformed state. Consequently, when it becomes necessary to detach the attachment member at a later time, the attachment member can be detached easily.

In a preferred embodiment of the above-described medical device of the present invention, the attachment member includes an adhesive material layer, a print layer, and an anti-slip layer that is formed of a resin material or a rubber material, and the adhesive material layer, the print layer, and the anti-slip layer are stacked in sequence.

With the above embodiment, a variety of patterns, figures, and characters can be printed on the attachment member. Accordingly, it is possible to customize the appearance of the medical device to be more suited to the preference of the user, so that the user can further deepen his or her attachment to the device. Furthermore, since the attachment member has an anti-slipping effect in the above-described embodiment, it is possible to suppress the occurrence of a situation where the user drops the medical device by accident.

In a preferred embodiment of the above-described medical device of the present invention, the attachment member includes an adhesive material layer, and an anti-slip layer having optical transparency and being formed of a resin material or a rubber material, the anti-slip layer includes granular identification members that are dispersed therein, and the identification members are formed of any of a luminescent material, a thermochromic material, and a light reflective material. In this case, the attachment member is prominent, which makes it possible to improve the identifiability of the medical device.

Furthermore, in another preferred embodiment of the above-described medical device of the present invention, the attachment member includes an adhesive material layer, an identification layer, and an anti-slip layer having optical transparency and being formed of a resin material or a rubber material, the adhesive material layer, the identification layer, and the anti-slip layer are stacked in sequence, and the identification layer is formed of a luminescent material or a light reflective material. Also in this case, the identifiability of the medical device can be improved with the attachment member.

Furthermore, in another preferred embodiment of the above-described medical device of the present invention, the attachment member includes an adhesive material layer, an anti-slip layer that is formed of a resin material or a rubber material, and an identification layer, the adhesive material layer, the anti-slip layer, and the identification layer are stacked in sequence, and the identification layer is formed of any of a luminescent material, a thermochromic material, and a light reflective material. Also in this case, the identifiability of the medical device can be improved with the attachment member.

Furthermore, in another embodiment of the above-described medical device of the present invention, the attachment member includes an adhesive material layer, a fragrance component-impregnated layer containing a fragrance component, and an anti-slip layer having porosity and being formed of a resin material or a rubber material, the fragrance component-impregnated layer is disposed on one side of the adhesive material layer, and the anti-slip layer is formed on the one side of the adhesive material layer so as to cover the fragrance component-impregnated layer. In this case, the medical device can relax the user with the fragrance component.

Furthermore, in another embodiment of the above-described medical device of the present invention, the attachment member includes an adhesive material layer, a circuit substrate on which a pressure sensor and a speaker are mounted, and an anti-slip layer that is formed of a resin material or a rubber material, the circuit substrate is disposed on one side of the adhesive material layer, and includes a driving circuit that drives the speaker in accordance with a signal from the pressure sensor, and the anti-slip layer is formed on the one side of the adhesive material layer so as to cover the circuit substrate, the pressure sensor, and the speaker. In this case, the user can confirm whether the medical device is supported to a sufficient degree from the sound.

Furthermore, in another embodiment of the above-described medical device of the present invention, the attachment member includes at least an adhesive material layer and an anti-slip layer that is formed of a resin material or a rubber material, and a recess is formed in the anti-slip layer. In this case, the grasping of the medical device by the user will be facilitated.

### Effects of the Invention

As described above, with the medical device according to the present invention, it is possible to alter the appearance at low cost without preventing the progress of size reduction.

### Brief Description of Drawings

[FIG. 1] FIG. 1 is a perspective view illustrating the configuration of a medical device according to an embodiment of the present invention, showing a state in which an attachment member is not attached to a casing of the medical device.
[FIG. 2] FIG. 2 is a perspective view illustrating the configuration of the medical device according to the embodiment of the present invention, showing a state in which an attachment member is attached to the casing of the medical device.
[FIG. 3] FIG. 3 is a cross-sectional view, taken along the cutting-plane line A-A' shown in FIG. 2.
[FIG. 4] FIG. 4 is a side view illustrating a medical device according to Embodiment 2 of the present invention.
[FIG. 5] FIG. 5 is a cross-sectional view of the medical device shown in FIG. 4 taken along the cutting-plane line B-B'.
[FIG. 6] FIG. 6 is a side view illustrating a medical device according to Embodiment 3 of the present invention.
[FIG. 7] FIG. 7 is a cross-sectional view of the medical device shown in FIG. 6 taken along the cutting-plane line C-C'.
[FIG. 8] FIG. 8 is a side view illustrating a medical device according to Embodiment 4 of the present invention.
[FIG. 9] FIG. 9 is a cross-sectional view of the medical device shown in FIG. 8 taken along the cutting-plane line D-D'.
[FIG. 10] FIG. 10 is an exploded perspective view illustrating a medical device according to Embodiment 5 of the present invention.
[FIG. 11] FIG. 11(a) is a cross-sectional view of the medical device shown in FIG. 10 taken along the cutting-plane line E-E', and FIG. 11(b) is a cross-sectional view of the medical device shown in FIG. 10 taken along the cutting-plane line F-F'.
[FIG. 12] FIGS. 12(a) and 12(b) are diagrams illustrating a second example of an attachment member that can be used for the medical device according to the present invention, wherein FIG. 12(a) is a plan view as viewed from the adhesive material layer side, and FIG. 12(b) is a cross-sectional view taken along the cutting-plane line G-G' in FIG. 12(a).
[FIG. 13] FIG. 13 is a perspective view illustrating a third example of an attachment member that can be used for the medical device according to the present invention.
[FIG. 14] FIG. 14 is a cross-sectional view illustrating a fourth example of an attachment member that can be used for the medical device according to the present invention.
[FIG. 15] FIG. 15 is a cross-sectional view illustrating a fifth example of an attachment member that can be used for the medical device according to the present invention.
[FIG. 16] FIG. 16 is a cross-sectional view illustrating a sixth example of an attachment member that can be used for the medical device according to the present invention.
[FIG. 17] FIG. 17 is a cross-sectional view illustrating a seventh example of an attachment member that can be used for the medical device according to the present invention.
[FIG. 18] FIG. 18 is a cross-sectional view illustrating an eighth example of an attachment member that can be used for the medical device according to the present invention.
[FIG. 19] FIG. 19 is a cross-sectional view illustrating a ninth example of an attachment member that can be used for the medical device according to the present invention.

### Description of Reference Numerals

- 1: medical device (Embodiment 1)
- 2: casing
- 3: attachment member
- 4: display screen
- 5: principal surface of casing
- 6, 7: surfaces (side surfaces) adjacent to principal surface
- 8: recess
- 8a: opening edge
- 9: anti-slip layer
- 10: print layer
- 11: adhesive material layer
- 12: gap
- 13: cover
- 14: elastic body
- 15: groove
- 16: wall portion
- 17: V-shaped portion
- 18: attachment member
- 19: attachment member
- 19a, 19b: through holes
- 20: medical device (Embodiment 2)
- 21: medical device (Embodiment 3)
- 22: medical device (Embodiment 4)
- 23: medical device (Embodiment 5)
- 24: rod member
- h: distance between groove and recess (thickness of wall portion)
- H: groove width
- i: groove depth
- I: recess depth

### Best Mode for Carrying Out the Invention

### Embodiment 1

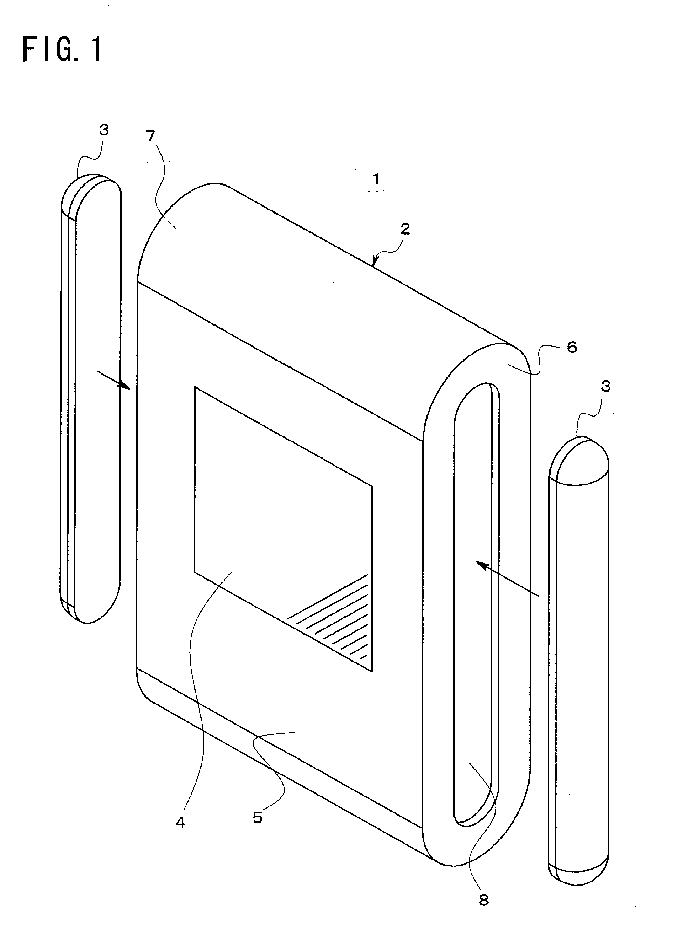

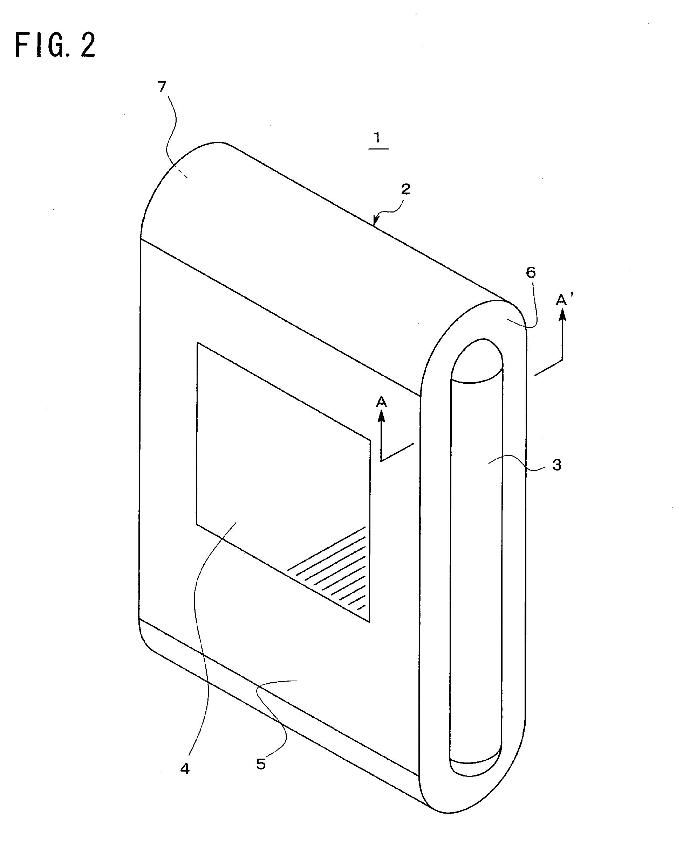

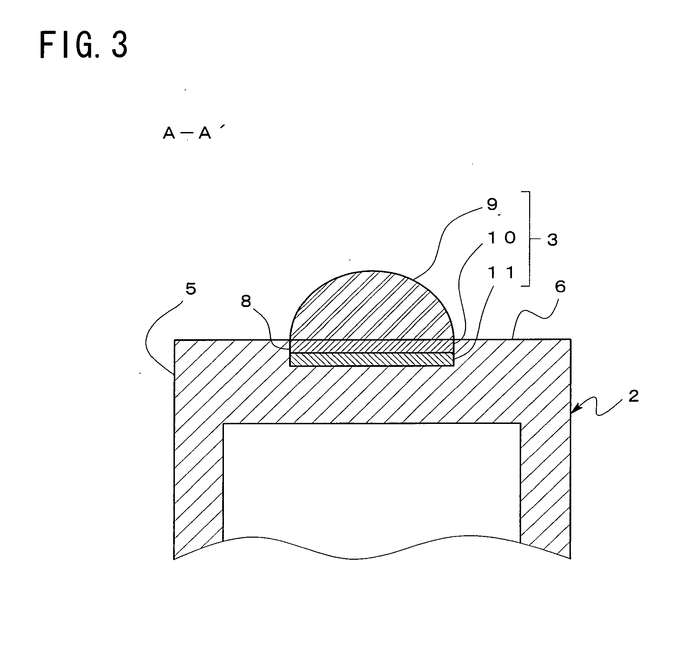

A medical device according to Embodiment 1 of the present invention will be described with reference to FIGS. 1 to 3. FIGS. 1 and 2 are perspective views illustrating the configuration of a medical device according to Embodiment 1 of the present invention. FIG. 1 shows a state in which an attachment member is not attached to a casing of the medical device, and FIG. 2 shows a state in which an attachment member is attached to the casing of the medical device. FIG. 3 is a cross-sectional view of the medical device shown in FIG. 2 taken along the cutting-plane line A-A'.

As shown in FIGS. 1 and 2, a medical device 1 according to Embodiment 1 is a medical device that measures a condition of a living body, including, for example, a glucose meter, a sphygmomanometer, a lactate meter, a ketone body measuring device, a thermometer, a urine analyzer, and a lipid measuring device. In addition, the medical device 1 is configured in a hand-held size, and is assumed to be carried by a user such as a patient.

The medical device 1 includes a casing 2. As shown in FIG. 1, the casing 2 is provided with a recess 8 on its exterior. In Embodiment 1, a display screen 4 that displays measurement results, including, for example, a blood glucose level and a blood pressure value is provided on one principal surface 5 of the exterior of the casing 2. Then, a recess 8 is formed in each of the surfaces (side surfaces) 6 and 7 that are adjacent to the principal surface 5. In addition, as shown in FIGS. 2 and 3, an attachment member 3 is attached to the inside of the recesses 8. It should be noted that the illustration of the recess formed in the external surface 7 has been omitted in FIG. 1.

Further, in Embodiment 1, the attachment member 3 can be formed by stacking an adhesive material layer 11, a print layer 10, and an anti-slip layer 9 in sequence, as shown in FIG. 3. However, there is no particular limitation with respect to the configuration of the attachment member 3 in Embodiment 1.

The anti-slip layer 9 can be formed of a resin material or a rubber material. Preferably, the resin material and the rubber material have a high coefficient of friction. Further, since the print layer 10 is provided in Embodiment 1, it is preferable that the resin material and rubber material have optical transparency. In the case where the print layer 10 is not provided, the resin material and rubber material may not have optical transparency. Further, the anti-slip layer 9 may be formed of a member whose coefficient of friction has been increased by forming recesses and projections on its surface, and a material other than the resin material or the rubber material may be used in this case.

Specific examples of the resin material for forming the anti-slip layer 9 include resin materials such as acrylate resins, urethane resins, polyethylene resins, polypropylene resins, ABS resins, polyvinyl chloride resins, AS resins, epoxy resins, and UV curable resins. Further, examples of the rubber material for forming the anti-slip layer 9 include rubber materials such as silicon rubbers, butadiene rubbers, isoprene rubbers, chloroprene rubbers, butyl-rubbers, fluorocarbon rubbers, and styrene-butadiene rubbers.

A variety of patterns, figures, characters and the like are printed on the print layer 10. In Embodiment 1, the printed patterns, figures, characters and the like can be visually recognized from the outside through the anti-slip layer 9. As the print layer 10, it is possible to use a polyethylene film, a polycarbonate film, paper, and the like.

There is no particular limitation with respect to the formation material, the thickness and the like of the adhesive material layer 11. As the formation material of the adhesive material layer 11, it is possible to use a material appropriately selected from the existing materials according to the formation material of the casing. Also, the thickness of the adhesive material layer 11 may be appropriately selected such that the desired adhesion can be achieved. For example, it is necessary to detach the attachment member 3 in the case where the anti-slip layer 9 has been deteriorated or damaged, or where the user wishes to alter the design of the print layer 10. Therefore, it is preferable that the adhesive material layer 11 is formed such that its adhesion will not be too strong.

By replacing the currently attached attachment member 3 with another attachment member 3 including a print layer 10 with a different pattern or an anti-slip layer 9 with a different color in this medical device 1, the appearance of the medical device 1 will be altered. With the medical device 1, the appearance can be altered without using the protective cover as in the case of conventional devices, so that it is possible to alter the appearance at low cost, and moreover, the size reduction of the medical device 1 will not be prevented.

In Embodiment 1, the attachment member 3 is attached to the casing 2 using the adhesive material layer 11. Accordingly, the user can easily change the attachment member 3, and therefore can customize the appearance of the medical device according to his or her preference, or enjoy variations in appearance. As a result, the user can deepen his or her attachment to the medical device 1, or feel refreshed. Further, if the attachment member 3 has a prominent design, then it is possible to prevent the medical device 1 from being lost, even if it is reduced in size and thus more susceptible to be lost.

Also, since the attachment member 3 can be provided with an anti-slipping effect in this embodiment, it is possible to prevent a situation where the user drops the device by accident. Furthermore, in Embodiment 1, the attachment member 3 is provided on the side surfaces, i.e., the surfaces 6 and 7, with which the hand of the user can most easily come into contact when the user operates the medical device 1. This enables the anti-slipping effect of the attachment member 3 to be exerted efficiently. Additionally, since the attachment member 3 is attached to the inside of the recess 8, it is possible to prevent the attachment member 3 from being detached as a result of coming into contact with another external object during use, unlike in the case where the attachment member 3 is simply attached to the exterior of the casing 2.

### Embodiment 2

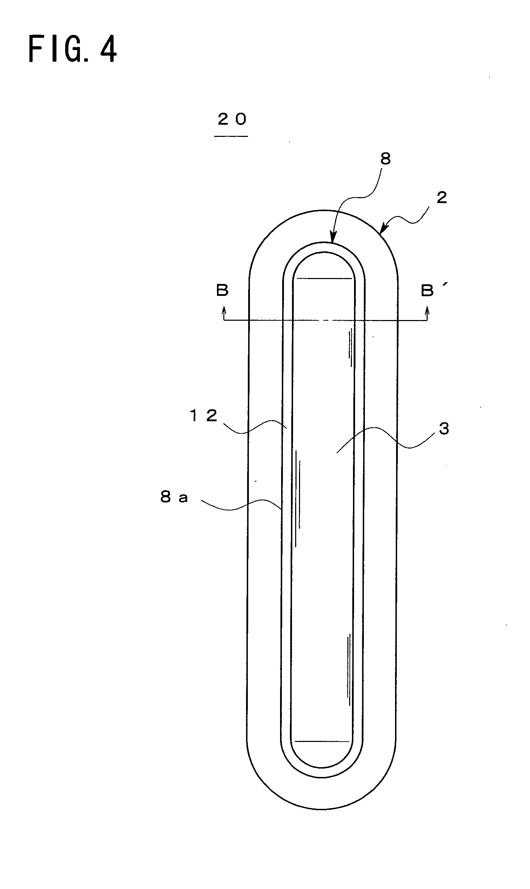

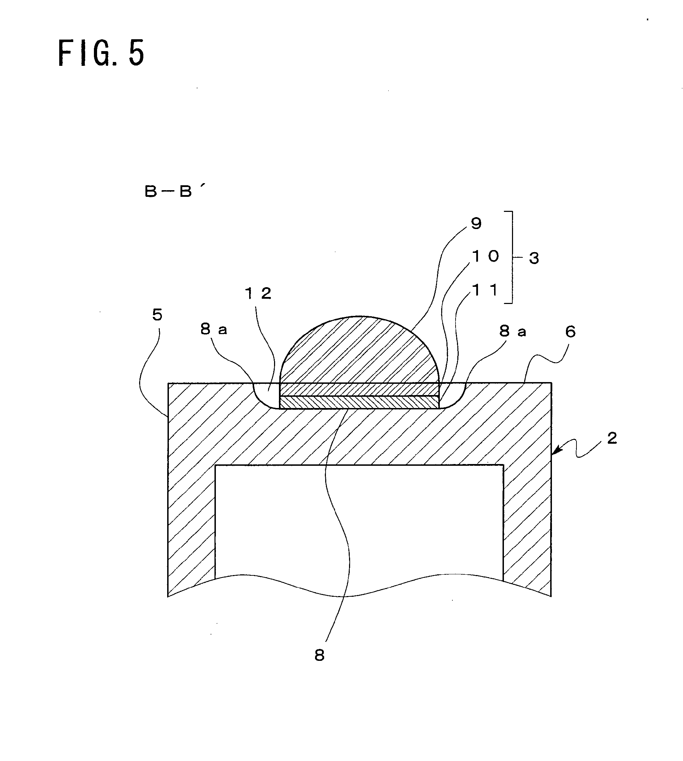

A medical device according to Embodiment 2 of the present invention will be described with reference to FIGS. 4 and 5. FIG. 4 is a side view illustrating the medical device according to Embodiment 2 of the present invention. FIG. 5 is a cross-sectional view of the medical device shown in FIG. 4 taken along the cutting-plane line B-B'.

As shown in FIGS. 4 and 5, in a medical device 20 according to Embodiment 2, a recess 8 is provided such that a gap 12 is formed between its opening edge 8a and an attachment member 3. In other words, the recess 8 is formed in such a manner that its opening area is larger than the attachment area of the attachment member 3.

In the example shown in FIGS. 4 and 5, the gap 12 is formed along the entire opening edge. As a result, when the medical device 20 is viewed from the side, a side groove is formed around the attachment member 3 (see FIG. 4). Except for the above-mentioned part, the medical device 20 is configured in the same manner as the medical device 1 according to Embodiment 1.

With this configuration, when using the medical device 20, the user can insert a finger, a fingernail, or the like into the gap 12, thereby easily detaching the attachment member 3. According to Embodiment 2, an old attachment member 3 can easily be detached when it is necessary to change the attachment member 3, and therefore the user can enjoy variations in appearance even more.

Here, if the gap 12 is too large, the attachment member 3 tends to easily come into contact with an external object, and detachment unintended by the user tends to occur. Therefore, the gap 12 needs to be formed so as not to be too large.

### Embodiment 3

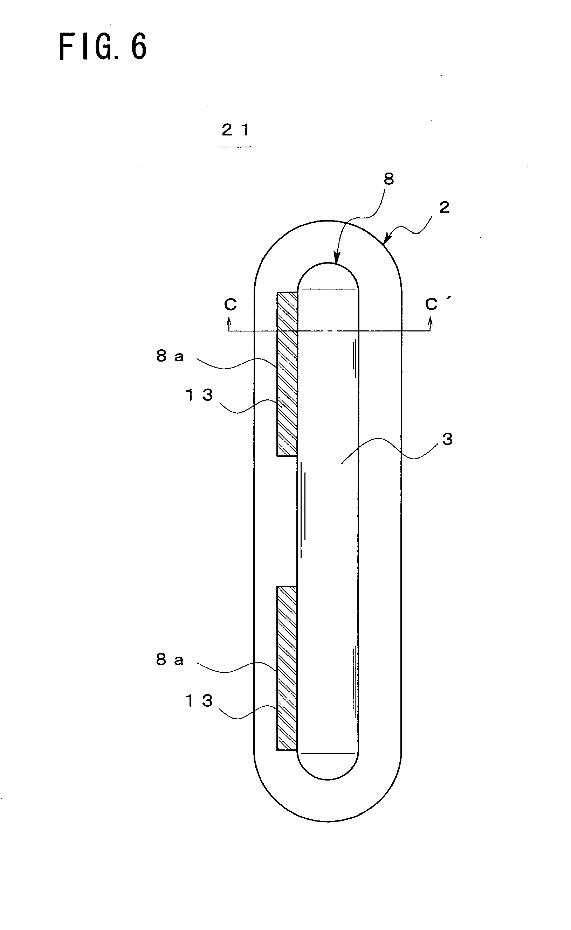

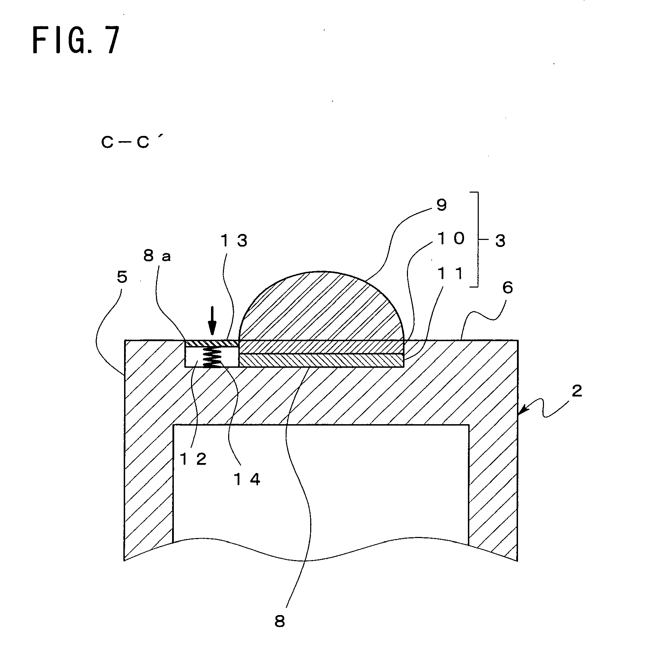

A medical device according to Embodiment 3 of the present invention will be described with reference to FIGS. 6 and 7. FIG. 6 is a side view illustrating the medical device according to Embodiment 3 of the present invention. FIG. 7 is a cross-sectional view of the medical device shown in FIG. 6 taken along the cutting-plane line C-C'.

As shown in FIGS. 6 and 7, as with the medical device 20 according to Embodiment 2, a medical device 21 according to Embodiment 3 is provided with a recess 8 formed such that a gap 12 is formed between its opening edge 8a and an attachment member 3.

However, unlike Embodiment 2, in Embodiment 3, the recess 8 is provided such that the gap 12 is formed only between a portion of the opening edge 8a and the attachment member 3. Additionally, unlike the medical device 20 according to Embodiment 2, the medical device 21 according to Embodiment 3 further includes a cover 13 that closes the gap 12. The cover 13 has a plate shape, and is fixed to the recess 8 via an elastic body 14. In FIG. 6, the cover 13 is hatched.

In the example shown in FIG. 7, a coil spring is used as the elastic body 14. One end of this coil spring (elastic body 14) is fixed to the cover 13, and the other end thereof is fixed to the bottom surface of the recess 8. Accordingly, the elastic body 14 is elastically deformed when the cover 13 is pressed toward the inside of the recess 8 by an external force (for example, when the user presses the cover 13 with a finger or the like). Then, when the external force exerted on the elastic body 14 is removed, the elastic body 14 causes the cover 13 to be restored to its original position.

Thus, unlike Embodiment 2, in Embodiment 3, the gap 12 is closed with the cover 13 in a normal condition. That is, until the user decides to detach the attachment member 3 and presses the cover 13, the presence of the gap 12 is concealed. Accordingly, with Embodiment 3, the occurrence of detachment of the attachment member 3 resulting from contact between the end of the attachment member 3 and an external object is suppressed as compared with Embodiment 2. It should be noted that except for the above-described part, the medical device 21 is configured in the same manner as the medical device 1 according to Embodiment 1 and the medical device 20 according to Embodiment 2.

### Embodiment 4

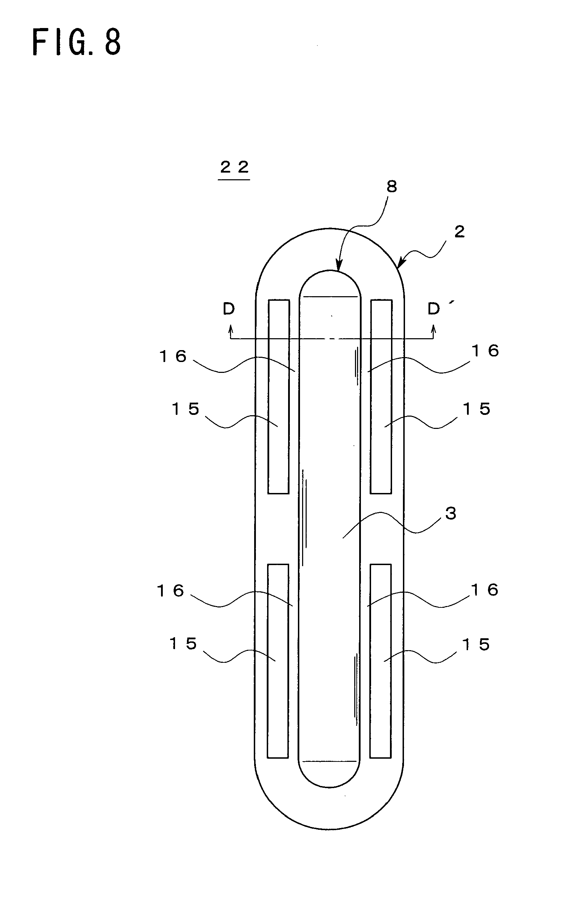

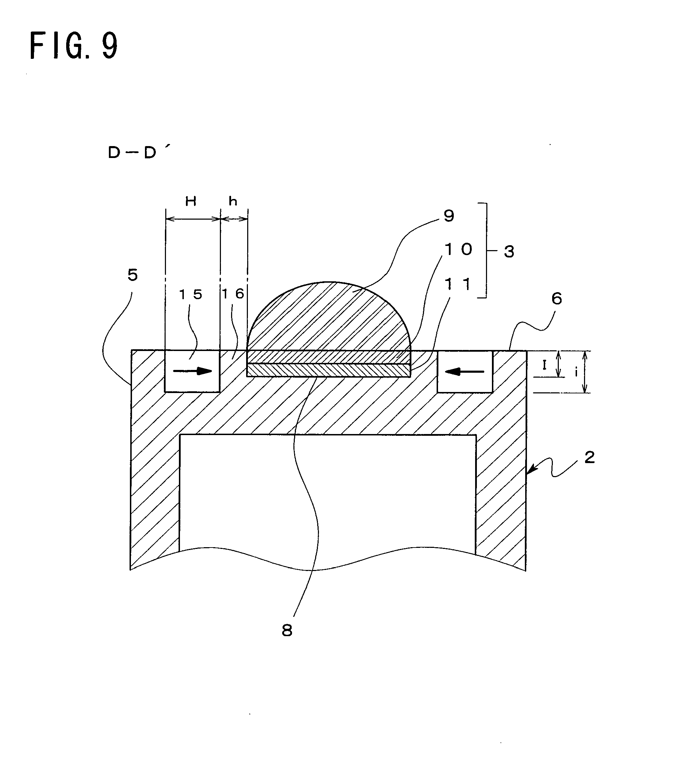

A medical device according to Embodiment 4 of the present invention will be described with reference to FIGS. 8 and 9. FIG. 8 is a side view illustrating the medical device according to Embodiment 4 of the present invention. FIG. 9 is a cross-sectional view of the medical device shown in FIG. 8 taken along the cutting-plane line D-D'.

As shown in FIGS. 8 and 9, in a medical device 22 according to Embodiment 4, a casing 2 is provided with grooves 15 along the outer circumference of a recess 8. In addition, the portion of the casing 2 that is located between the grooves 15 and the recess 8 serves as a wall portion 16 facing an attachment member 3.

With this configuration, in Embodiment 4, the user can press the wall portion 16 from the inside of the grooves 15 toward the attachment member 3 (in the directions indicated by the arrows in FIG. 9). Then, this pressing causes the attachment member 3 to be deformed, so that the area of adhesion of the adhesive material layer 11 to the recess 8 is reduced, and the attachment member 3 becomes easily detached.

In the example shown in FIGS. 8 and 9, two grooves 15 are provided along each longitudinal edge of the attachment member 3. Therefore, the grooves 15 adjacent to one longitudinal edge are opposed to the grooves 15 adjacent to the other longitudinal edge, so that the attachment member 3 is surrounded by the wall portion 16. Accordingly, the user can pinch the attachment member 3 between, for example, the thumb and the forefinger via the wall portion 16, thereby easily deforming the attachment member 3.

Thus, with Embodiment 4, it is possible to perform the operation for detaching the attachment member 3 more easily than with Embodiment 1. Further, since the end of the attachment member 3 does not easily become exposed to the outside, it is also possible to suppress the occurrence of detachment of the attachment member 3 resulting from contact between the end of the attachment member 3 and an external object. It should be noted that except for the above-described part, the medical device 22 is configured in the same manner as the medical device 1 according to Embodiment 1.

Further, in Embodiment 4, in order to facilitate the pressing of the attachment member 3 via the wall portion 16, it is preferable to use the following resins or rubbers as the formation material of the casing 2. Examples of the resins include polyethylene terephthalate resins, vinyl chloride resins, phenol resins, nylon amide resins, polyimide resins, polyetherimide resins, and polysulfone resins. Examples of the rubbers include silicon rubbers, butadiene rubbers, isoprene rubbers, chloroprene rubbers, butyl-rubbers, fluorocarbon rubbers, and styrene-butadiene rubbers.

Additionally, it is preferable that the depth i of the grooves 15 is, for example, two to three times the depth I of the recess 8. Furthermore, it is preferable that the distance h between the grooves 15 and the recess 8 (the thickness of the wall portion 16) is, for example, 0.3 times to 0.5 times the width H of the grooves 15.

### Embodiment 5

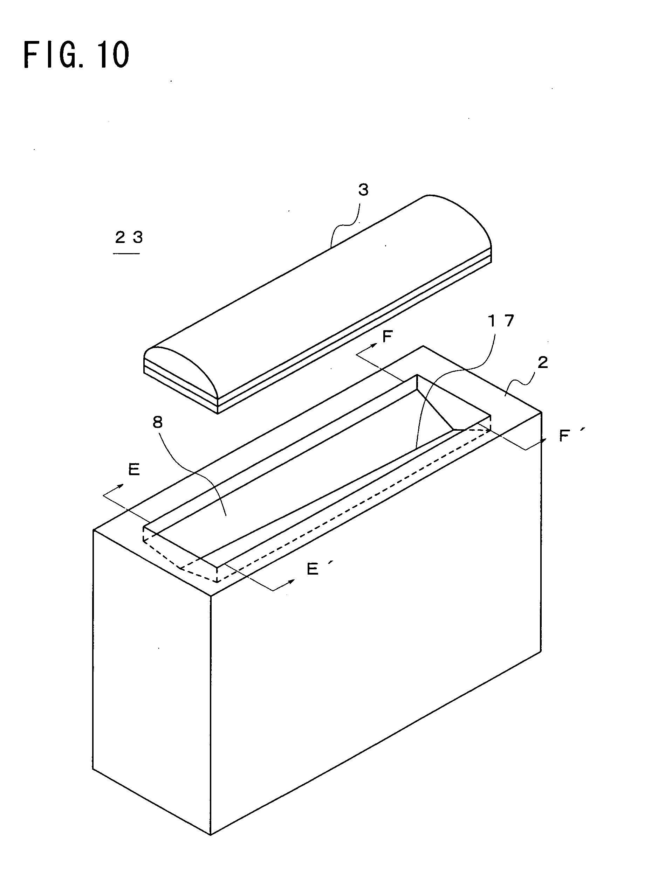

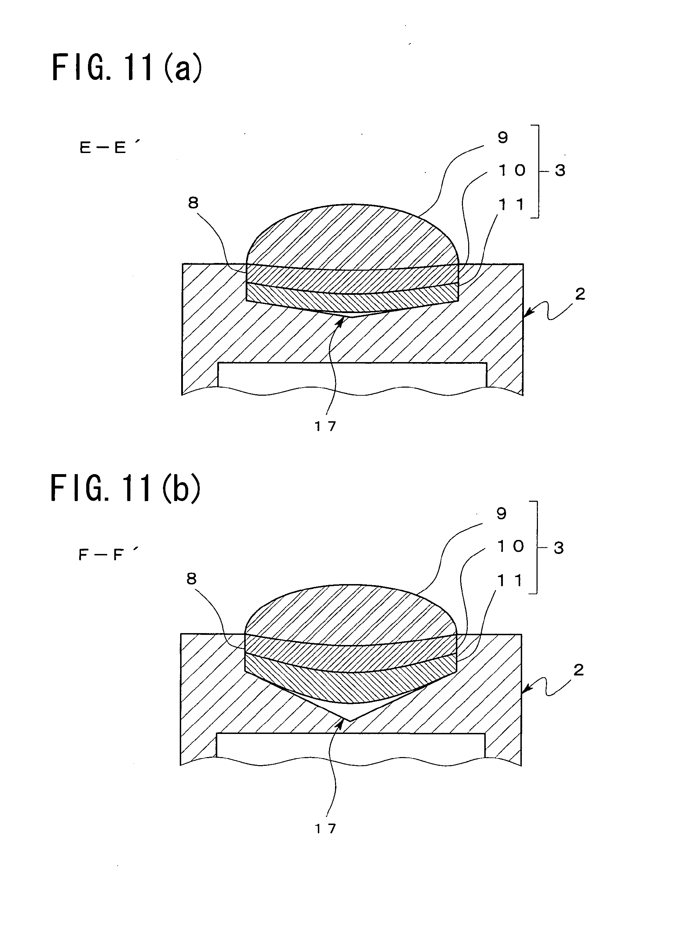

A medical device according to Embodiment 5 of the present invention will be described with reference to FIGS. 10 and 11. FIG. 10 is an exploded perspective view illustrating the medical device according to Embodiment 5 of the present invention. FIG. 11(a) is a cross-sectional view of the medical device shown in FIG. 10 taken along the cutting-plane line E-E', and FIG. 11(b) is a cross-sectional view of the medical device shown in FIG. 10 taken along the cutting-plane line F-F'.

As shown in FIGS. 10, 11(a) and 11(b), in a medical device 23 according to Embodiment 5, a recess 8 has been formed such that the bottom of a cross section thereof is V-shaped. Accordingly, an attachment member 3 is attached to the inside of the recess 8 in an elastically deformed state, and a portion of the adhesive surface of an adhesive material layer 11 of the attachment member 3 is not in contact with the inside of the recess 8. Consequently, when it becomes necessary to detach the attachment member 3 at a later time, the user can easily detach the attachment member 3. Further, the degree of detachability can be set with the angle of a V-shaped portion 17 in the manner described below.

In Embodiment 5, the recess 8 is formed such that the angle of its V-shaped portion 17 varies, as shown in FIGS. 10, 11(a) and 11(b). Specifically, the angle of the V-shaped portion 17 decreases from the cross section taken along the cutting-plane line E-E' toward the cross section taken along the cutting-plane line F-F'. Accordingly, the area of adhesion of the attachment member 3 to the recess 8 is not constant, and varies from the cross section taken along the cutting-plane line E-E' toward the cross section taken along the cutting-plane line F-F'.

That is, in Embodiment 5, the adhesion of the attachment member 3 decreases toward the cross section taken along the cutting-plane line F-F'. Embodiment 5 is effective when it is desired to control the adhesion of the attachment member 3 on a portion-by-portion basis. Furthermore, in this way, the attachment member 3 can also be attached to a portion whose bottom surface is not flat.

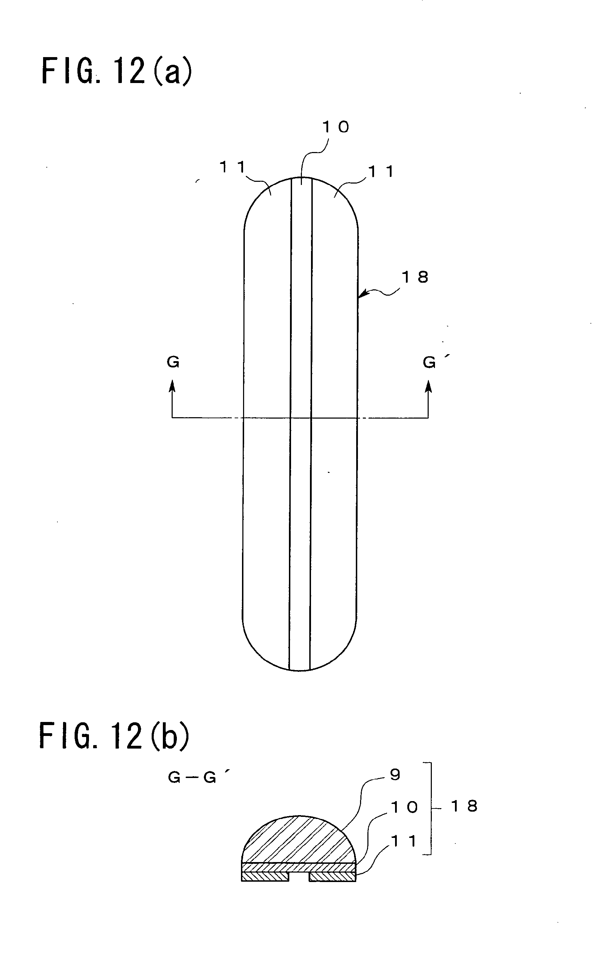

In addition, the medical device according to the present invention may also be embodied differently from the embodiments described in Embodiments 1 to 5 above. For example, it is also possible to use the attachment members shown in FIGS. 12 to 19 in place of the attachment member 3. Here, the attachment member 3 shown in FIGS. 3, 5, 7, 9, 11(a) and 11(b) in Embodiments 1 to 5 is taken as a first example, the attachment members shown in FIGS. 12 to 19 are taken as second to ninth examples, respectively, and the attachment members of the second to ninth examples will be described hereinafter.

FIGS. 12(a) and 12(b) are diagrams illustrating the second example of an attachment member that can be used for the medical device according to the present invention, wherein FIG. 12(a) is a plan view as viewed from the adhesive material layer side, and FIG. 12(b) is a cross-sectional view taken along the cutting-plane line G-G' in FIG. 12(a).

Unlike the example shown in FIG. 3, the adhesive material layer 11 is not formed across the entire surface of the print layer 10 in an attachment member 18 shown in FIGS. 12(a) and 12(b). The adhesion of the adhesive material layer 11 in the attachment member 18 is weaker than the adhesion of the adhesive material layer 11 of the attachment member 3 shown in FIG. 3. That is, in the second example, the adhesion is weakened by reducing the area of adhesion. The second example is effective in the case where the attachment member is frequently detached.

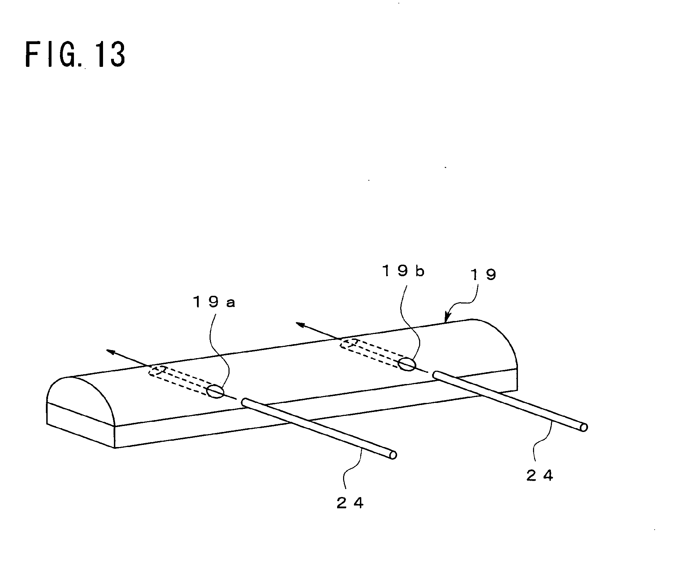

FIG. 13 is a perspective view illustrating the third example of an attachment member that can be used for the medical device according to the present invention. In an attachment member 19 shown in FIG. 13, through holes 19a and 19b are formed so as to penetrate through the attachment member 19. Accordingly, the attachment member 19 can be easily detached, for example, by inserting a rod member 24 through each of the through holes 19a and 19b, and using the two rod members 24 as holding handles. By using the attachment member 19 of the third example shown in FIG. 13, the ease of changing the attachment member 19 by the user can be facilitated.

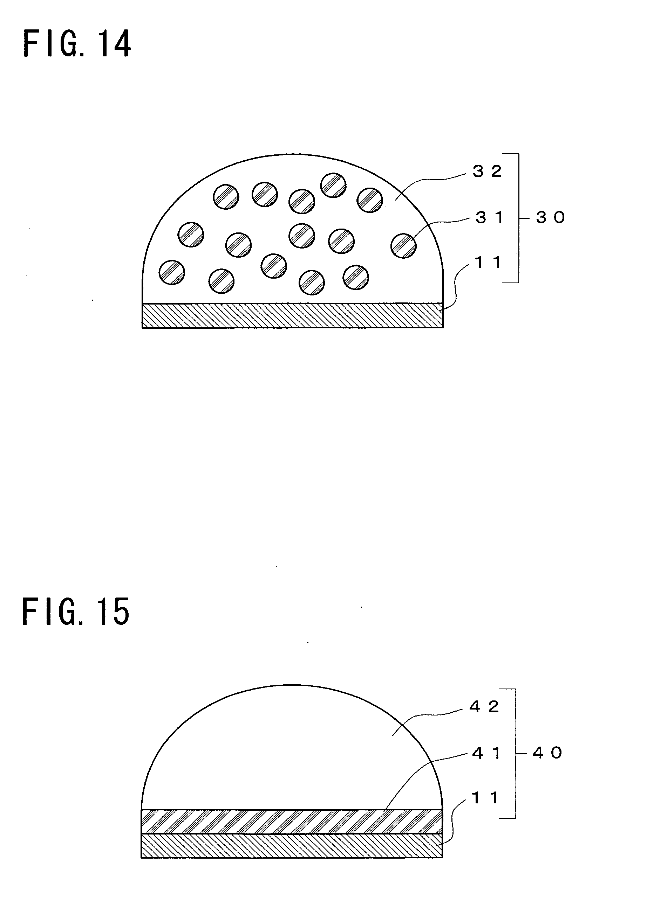

FIG. 14 is a cross-sectional view illustrating the fourth example of an attachment member that can be used for the medical device according to the present invention. As shown in FIG. 14, an attachment member 30 of the fourth example includes an adhesive material layer 11 and an anti-slip layer 32. In addition, granular identification members 31 are dispersed in the anti-slip layer 32. Also, the identification members 31 are formed of a luminescent material, a thermochromic material, a light reflective material, or the like. It should be noted that the hatching of the anti-slip layer 32 has been omitted in FIG. 14.

Here, examples of the luminescent material include a phosphorescent material and a fluorescent material. Specific examples of the phosphorescent material include a material obtained by adding a rare-earth element such as europium (Eu) or dysprosium (Dy) to a crystal matrix formed of strontium aluminate (SrAl₂O₄), and a material obtained by adding copper (Cu) to zinc sulfide (ZnS). Examples of the fluorescent material include fluorescein isothiocyanate and tetramethyl rhodamine isothiocyanate.

Further, examples of the thermochromic material include a material containing a color former such as a leuco dye, a developer, and a color-changing-temperature controlling agent, wherein the color former and the developer bind to each other when the temperature has reached a certain temperature, thereby forming a color. A specific example thereof is metamo color (registered trademark) manufactured by PILOT Corporation. Examples of the reflective material include metallic materials and glass materials.

The anti-slip layer 32 can be formed using the same formation material as the formation material of the anti-slip layer 9 shown in FIG. 3. However, in the fourth example, the anti-slip layer 32 is required to have optical transparency so as to allow light to incident on the identification members 31 from the outside. In addition, the adhesive material layer 11 is the same as the adhesive material layer 11 shown in FIG. 3.

With this configuration, the attachment member 30 is highly visible from the outside, and therefore, applying the attachment member 30 to a medical device allows patients, in particular, patients with weak eyesight to identify their own medical devices easily. Furthermore, in the case of using the above-described thermochromic material as the formation material of the identification members 31, the identification members 31 detect the body temperature of the user or the ambient temperature, and changes color. Then, by setting the temperatures at which the thermochromic material begins to change color to the upper limit (e.g., 40°C) and the lower limit (e.g., 10°C) of the usable temperature of the medical device, the user can determine whether the medical device is at a usable temperature based on the color of the identification members 31.

FIG. 15 is a cross-sectional view illustrating the fifth example of an attachment member that can be used for the medical device according to the present invention. As shown in FIG. 15, an attachment member 40 of the fifth example includes an identification layer 41 between an adhesive material layer 11 and an anti-slip layer 42. The identification layer 41 is formed of a luminescent material, a light reflective material, or the like. It should be noted that the hatching of the anti-slip layer 42 has been omitted in FIG. 15.

Here, the luminescent materials described in the fourth example can be used as the luminescent material. However, in the fifth example, the luminescent material is shaped in the form of a layer. Further, examples of the light reflective material include metallic materials and retro-reflective materials. Specific examples of the metallic materials include aluminum and silver, and these materials are shaped into a foil or a thin film. Further, specific examples of the retro-reflective material include a sheet material in which a large number of glass beads having a diameter of approximately 50 µm to 500 µm are arranged on its surface.

Also in the fifth example, the anti-slip layer 42 is required to have optical transparency for allowing light to incident on the identification layer 41 from the outside as with the fourth example. However, except for this, the anti-slip layer 42 can be formed in the same manner as the anti-slip layer 9 shown in FIG. 3. Further, the adhesive material layer 11 is the same as the adhesive material layer 11 shown in FIG. 3. With this configuration, the attachment member 40 is also highly visible from the outside, and therefore, applying the attachment member 40 to a medical device also allows patients, in particular, patients with weak eyesight to identify their own medical devices easily.

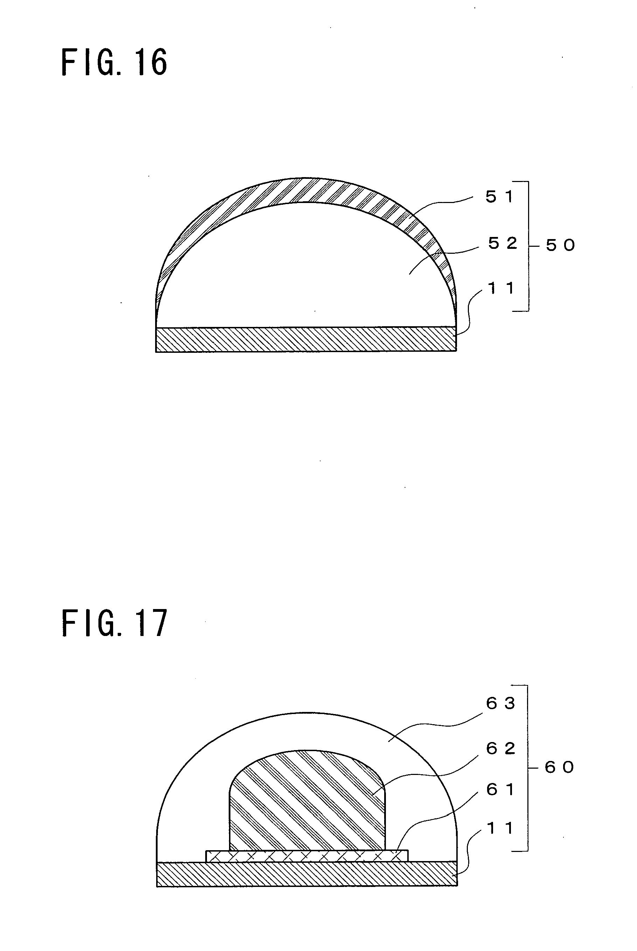

FIG. 16 is a cross-sectional view illustrating the sixth example of an attachment member that can be used for the medical device according to the present invention. As shown in FIG. 16, an attachment member 50 is formed by stacking an adhesive material layer 11, an anti-slip layer 52, and an identification layer 51 in sequence. The identification layer 51 can be formed in the same manner as the identification layer 41 of the fourth example shown in FIG. 14. It should be noted that the hatching of the anti-slip layer 52 has been omitted in FIG. 16.

Furthermore, in the sixth example, the identification layer 51 may also be formed using the thermochromic material discussed in the description of the fourth example shown in FIG. 14. In this case, the identification layer 51 detects the body temperature of the user or the ambient temperature, and changes color as with the fourth example. Then, by setting the temperatures at which the thermochromic material begins to change color to the upper limit and the lower limit of the usable temperature of the medical device, the user can determine whether the medical device is at a usable temperature based on the color of the identification members 31.

In the sixth example, there is no particular limitation with respect to the degree of optical transparency in the anti-slip layer 52, and the same anti-slip layer as the anti-slip layer 9 shown in FIG. 3 can be used as the anti-slip layer 52. The adhesive material layer 11 is the same as the adhesive material layer 11 shown in FIG. 3. In the sixth example, the identification layer 51 is located at the uppermost layer, and therefore, applying the attachment member 50 to a medical device further enhances the ease of identification by the user (in particular, patients with weak eyesight).

Further, the sixth example may be embodied such that it includes a sheet-like thermometer attached in place of the identification layer 51. Specific examples of the sheet-like thermometer include a liquid crystal thermometer including a cholesteric liquid crystal that changes color depending on the temperature. The liquid crystal thermometer can inform the user of approximate temperatures such as 10°C, 15°C, 20°C, 25°C, 30°C, 35°C, and 40°C. Therefore, the user can determine whether the medical device is in the usable temperature range also in this case, as in the case where the identification layer 51 is formed using a thermochromic material.

FIG. 17 is a cross-sectional view illustrating the seventh example of an attachment member that can be used for the medical device according to the present invention. Unlike the attachment members of the first to sixth examples, an attachment member 60 of the seventh example shown in FIG. 17 has the function of relaxing the user. As shown in FIG. 17, the attachment member 60 includes an adhesive material layer 11, a separation layer 61, a fragrance component-impregnated layer 62, and an anti-slip layer 63. The separation layer 61 and the fragrance component-impregnated layer 62 are stacked in sequence on one side of the adhesive material layer 11, and the anti-slip layer 63 is further formed on the one side of the adhesive material layer 11 so as to cover the separation layer 61 and the fragrance component-impregnated layer 62. It should be noted that the hatching of the anti-slip layer 63 has been omitted in FIG. 17.

The fragrance component-impregnated layer 62 can be formed by impregnating a porous material with a material containing a fragrance component. Examples of the porous material include porous materials obtained by using the resin materials and rubber materials described as the formation materials of the anti-slip layer 9 of the first example as the starting materials, and foaming these materials.

Examples of the material containing a fragrance component include orange oil, lemon oil, lime oil, petitgrain oil , citrus junos oil, Neroli oil, bergamot oil, lavender oil, lavandin oil, abies oil, bay oil, ylang-ylang oil, sandalwood oil, labdanum oil, citronella oil, geranium oil, peppermint oil, peppermint oil, spearmint oil, eucalyptus oil, lemongrass oil, patchouli oil, jasmine oil, rose oil, cedar oil, vetiver oil, galbanum oil, oakmoss oil, pine oil, camphor oil, wood sandal oil, linalool, geraniol, citronellol, 1-menthol, borneol, β -phenylethyl alcohol, n-nonyl aldehyde, citral, cinnamic aldehyde, lilial, vanillin, camphor, ionone, ethylene brassylate, galaxolid, and linalyl acetate.

The anti-slip layer 63 may be any anti-slip layer that allows passage of the fragrance component contained in the fragrance component-impregnated layer 62, and specifically, the above-described porous materials can also be used as the anti-slip layer 63. However, the anti-slip layer 63 is a part to which the hand of the user comes into direct contact, and therefore preferably has some degree of flexibility. Specifically, it is preferable to use a porous material having a pore diameter of about 300 µm to 3000 µm as the porous material for forming the anti-slip layer 63.

The separation layer 61 is disposed so as to prevent a reaction between the fragrance component contained in the fragrance component-impregnated layer 62 and the adhesive contained in the adhesive material layer 11. As the separation layer 61, it is possible to use metal foils such as an aluminum foil. The adhesive material layer 11 is the same as the adhesive material layer 11 shown in FIG. 3.

With this configuration, when the user holds a medical device to which the attachment member 60 is attached, the fragrance component diffuses from the attachment member 60. Then, the user can be relaxed with the fragrance component. In particular, when the user is a patient suffering from a disease, the fragrance component can be expected to alleviate pain. Furthermore, if the user is allowed to select the fragrance, the user can identify his or her own medical device by the fragrance from the attachment member 60.

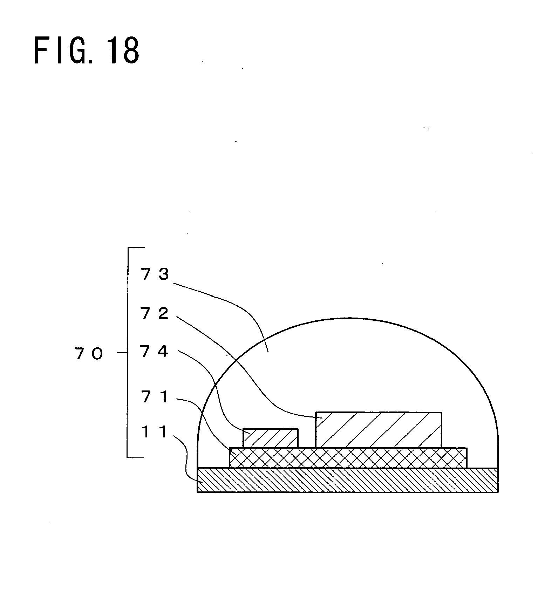

FIG. 18 is a cross-sectional view illustrating the eighth example of an attachment member that can be used for the medical device according to the present invention. Unlike the attachment members of the first to seventh examples, an attachment member 70 of the eighth example shown in FIG. 18 has the function of emitting a sound when subjected to pressure. As shown in FIG. 18, the attachment member 70 includes an adhesive material layer 11, a circuit substrate 71, and an anti-slip layer 73. A pressure sensor 72 and a thin speaker 74 are mounted on the circuit substrate 71. It should be noted that the hatching of the anti-slip layer 73 has been omitted in FIG. 18.

The circuit substrate 71 includes a driving circuit disposed on one side of the adhesive material layer 11 for driving the speaker 74, and a power supply circuit for supplying power to the speaker 74 (these circuits are not shown). In addition, the power supply circuit is also provided with a battery (not shown). The anti-slip layer 73 is formed such that it covers the circuit substrate 71, the pressure sensor 72, and the speaker 74.

The pressure sensor 72 may be any pressure sensor that can detect pressing force applied to the anti-slip layer 73. In the example shown in FIG. 18, the pressure sensor 72 includes a diaphragm (not shown) on which a piezoresistive element is formed. Accordingly, when the anti-slip layer 73 is pressed, and thereby the diaphragm is deformed, the electric resistance of the piezoresistive element changes, and at the same time, the level of the electric signal from the pressure sensor 72 also changes.

The driving circuit (not shown) included in the circuit substrate 71 has the function of detecting the level of the electric signal from the pressure sensor 72, and detects pressing of the anti-slip layer 73. Then, upon detection of pressing of the anti-slip layer 73, the driving circuit causes the power supply circuit to supply power to the speaker 74, thereby outputting a predetermined sound from the speaker 74.

As the anti-slip layer 73, it is possible to use the same anti-slip layer as the anti-slip layer 9 shown in FIG. 3. Further, as with the anti-slip layer 63 shown in FIG. 17, a porous material can also be used as the anti-slip layer 73. The adhesive material layer 11 is the same as the adhesive material layer 11 shown in FIG. 3.

With this configuration, when the user holds a medical device to which the attachment member 70 is attached, a sound is output from the attachment member 70. The attachment member 70 of the eighth example is particularly effective for a patient with a reduced sense of touch in his or her fingertips. Further, it is possible to set a threshold for the pressure applied to the anti-slip layer 73 in the attachment member 70, and no sound may be output if the pressure does not reach the set threshold. In this case, the patient can recognize that the medical device is securely held based on the sound from the attachment member 70. Furthermore, this can also lead to a training for enhancing the grasping ability of the patient.

Further, by allowing the user to select the sound that is output, the user can identify his or her own medical device based on the sound from the attachment member 70. Furthermore, by outputting music from the attachment member 70, the user can be relaxed by the music. In particular, when the user is a patient suffering from a disease, the music can be expected to alleviate pain. In addition, a medical device to which the attachment member 70 is attached also outputs a sound when the user has dropped the device by accident. Accordingly, this is particularly effective for the user with hearing loss or blindness.

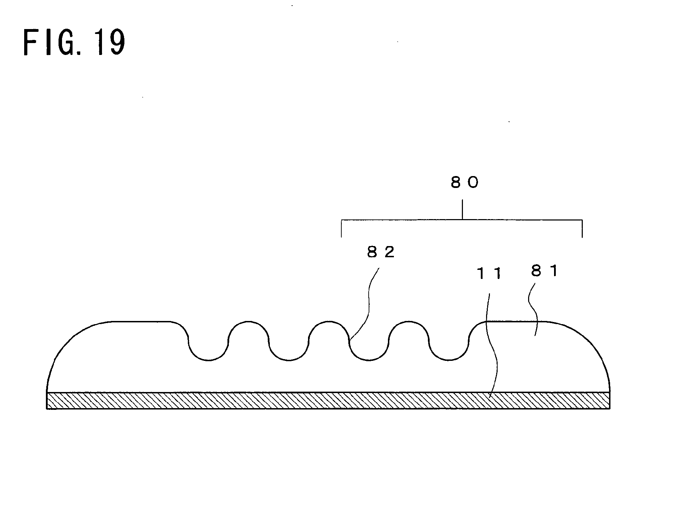

FIG. 19 is a cross-sectional view illustrating the ninth example of an attachment member that can be used for the medical device according to the present invention. The cross section shown in FIG. 19 is a cross section of an attachment member 80 along its major axis direction. Unlike the attachment members of the first to eighth examples, the attachment member 80 of the ninth example shown in FIG. 19 includes a recess 82 for facilitating grasping by the user. Specifically, as shown in FIG. 19, the attachment member 80 includes an adhesive material layer 11 and an anti-slip layer 81 that is disposed on one side of the adhesive material layer 11. Four recesses 82 are formed in the anti-slip layer 81. It should be noted that the hatching of the anti-slip layer 81 has been omitted in FIG. 19.

The recesses 82 are formed along the minor axis of the attachment member 80 with spaces therebetween so that the fingers of the user are fit therein when the user grasps the medical device to which the attachment member 80 is attached. The number, width, depth, and the like of the recesses 82 may be appropriately set, without any particular limitations. The anti-slip layer 81 in which the recesses 82 are formed can be formed by injecting the same formation material as the formation material of the anti-slip layer 9 shown in FIG. 3 into a mold, and curing the material.

Furthermore, in the case of conforming the arrangement and the shape of the recesses 82 to the shape of the hand of the user, the anti-slip layer 81 may be formed by the following procedure. First, a cast of the shape of the hand of the user is made using a silicon rubber or the like, and a male mold is produced from this cast. Next, by producing a female mold from this male mold, and injecting the formation material into the female mold, an anti-slip layer 81 whose arrangement and shape of the recesses 82 conform to the shape of the hand of the user is formed. Further, a porous material can also be used as the anti-slip layer 81 as with the anti-slip layer 63 shown in FIG. 17. The adhesive material layer 11 is the same as the adhesive material layer 11 shown in FIG. 3.

With this configuration, a medical device to which the attachment member 80 is attached can be easily held by the user. As with the attachment member 70 of the eighth example, the attachment member 80 of the ninth example is particularly effective when the user is a patient with a reduced sense of touch in his or her fingertips. Further, since the user can securely hold the medical device based on the sense of touch, this configuration is also effective when the user has weak eyesight.

Incidentally, each of the medical devices shown in Embodiments 1 to 5 described above also has a unique function, and can meet various user preferences. Accordingly, the medical devices shown in Embodiments 1 to 5 are required to be sold in a state where they have been customized according to the user's request.

Specifically, a sales system is required by which the user can display a customized virtual medical device on the screen of the user's own computer device, and can purchase the displayed medical device. Such a sales system can be provided using a server device that provides a sales website. The user may access the server device using his or her own terminal device via the Internet. With this sales system, the user can customize the type of the attachment member (see FIGS. 3, and 12 to 19); the shape of the casing recess, the casing color, and the like, thereby obtaining a medical device specially made for the user.

### Industrial Applicability

As described above, the present invention is effective for altering the appearance of a medical device, in particular, a small portable medical device. The medical device according to the present invention has industrial applicability.

## Claims

1. A medical device that measures a condition of a living body, comprising:
a casing; and
an attachment member,
wherein the attachment member is attached to an exterior of the casing.

2. The medical device according to claim 1,
wherein a recess is provided in the exterior of the casing, and
the attachment member is attached to the inside of the recess.

3. The medical device according to claim 2,
wherein the casing is provided with a display screen that displays a measurement result, and
the recess is provided in a surface adjacent to a surface where the display screen is provided.

4. The medical device according to claim 2,
wherein the recess is provided such that a gap is formed between an opening edge thereof and the attachment member.

5. The medical device according to claim 4,
wherein the medical device further comprises a cover that closes the gap,
the cover is fixed to the recess via an elastic body, and
the elastic body is elastically deformed when the cover is pressed toward the inside of the recess by an external force.

6. The medical device according to claim 2,
wherein the casing is provided with a groove along an outer circumference of the recess.

7. The medical device according to claim 2,
wherein the recess is formed such that the bottom of a cross section thereof is V-shaped.

8. The medical device according to any of claims 1 to 7,
wherein the attachment member comprises an adhesive material layer, a print layer, and an anti-slip layer that is formed of a resin material or a rubber material, and
the adhesive material layer, the print layer, and the anti-slip layer are stacked in sequence.

9. The medical device according to any of claims 1 to 7,
wherein the attachment member comprises an adhesive material layer, and an anti-slip layer having optical transparency and being formed of a resin material or a rubber material,
the anti-slip layer includes granular identification members that are dispersed therein, and
the identification members are formed of any of a luminescent material, a thermochromic material, and a light reflective material.

10. The medical device according to any of claims 1 to 7,
wherein the attachment member comprises an adhesive material layer, an identification layer, and an anti-slip layer having optical transparency and being formed of a resin material or a rubber material,
the adhesive material layer, the identification layer, and the anti-slip layer are stacked in sequence, and
the identification layer is formed of a luminescent material or a light reflective material.

11. The medical device according to any of claims 1 to 7,
wherein the attachment member comprises an adhesive material layer, an anti-slip layer that is formed of a resin material or a rubber material, and an identification layer,
the adhesive material layer, the anti-slip layer, and the identification layer are stacked in sequence, and
the identification layer is formed of any of a luminescent material, a thermochromic material, and a light reflective material.

12. The medical device according to any of claims 1 to 7,
wherein the attachment member comprises an adhesive material layer, a fragrance component-impregnated layer containing a fragrance component, and an anti-slip layer having porosity and being formed of a resin material or a rubber material,
the fragrance component-impregnated layer is disposed on one side of the adhesive material layer, and
the anti-slip layer is formed on the one side of the adhesive material layer so as to cover the fragrance component-impregnated layer.

13. The medical device according to any of claims 1 to 7,
wherein the attachment member comprises an adhesive material layer, a circuit substrate on which a pressure sensor and a speaker are mounted, and an anti-slip layer that is formed of a resin material or a rubber material,
the circuit substrate is disposed on one side of the adhesive material layer, and comprises a driving circuit that drives the speaker in accordance with a signal from the pressure sensor, and
the anti-slip layer is formed on the one side of the adhesive material layer so as to cover the circuit substrate, the pressure sensor, and the speaker.

14. The medical device according to any of claims 1 to 7,
wherein the attachment member comprises at least an adhesive material layer and an anti-shp layer that is formed of a resin material or a rubber material, and
a recess is formed in the anti-slip layer.
